# EUROPÄISCHE PATENTSCHRIFT

(11) **EP 0 950 404 B1**
(45) Veröffentlichungstag und Bekanntmachung des Hinweises auf die Patenterteilung: **16.06.2004**
(21) Anmeldenummer: 99105799.3
(22) Anmeldetag: 22.03.1999
(51) Int. Cl.: A61K 9/22, A61K 47/32

(54) **Verfahren zur Herstellung von festen Dosierungsformen**
Process for the preparation of solid dosage forms
Procédé de préparation de formes solides de dosage

(30) Priorität: 23.03.1998 DE 19812688
(43) Veröffentlichungstag der Anmeldung: 20.10.1999
(73) Patentinhaber: BASF AKTIENGESELLSCHAFT, 67056 Ludwigshafen (DE)
(72) Erfinder: Kothrade, Stephan, 67117 Limburgerhof (DE); Berndl, Gunther, 67273 Herxheim (DE); Simon, Dirk, 67112 Mutterstadt (DE); Sanner, Axel, 67227 Frankenthal (DE)
(74) Vertreter: Kinzebach, Werner, Dr.

(56) Entgegenhaltungen:
- EP-A- 0 240 904
- EP-A- 0 544 144
- WO-A-95/24430
- CHEN, GUOHUA ET AL: "Temperature-induced gelation Pluronic -g-poly( acrylic acid) graft copolymers for prolonged drug delivery to the eye" ACS SYMP. SER. (1997), 680(POLY(ETHYLENE GLYCOL)), 441-457 , XP002120272

## Beschreibung

Die Erfindung betrifft ein Verfahren zur Herstellung von festen Dosierungsformen durch Vermischen von mindestens einem polymeren Bindemittel und gegebenenfalls mindestens einem Wirkstoff und gegebenenfalls üblichen Additiven unter Bildung eines plastischen Gemisches und Formgebung. Insbesondere betrifft die Erfindung ein Verfahren zur Herstellung von festen pharmazeutischen Formen.

Die klassischen Verfahren zur Herstellung fester pharmazeutischer Formen, insbesondere Tabletten, werden diskontinuierlich durchgeführt und umfassen mehrere Stufen. Pharmazeutische Granulate stellen hierbei ein wichtiges Zwischenprodukt dar. So ist z. B. dem Buch "Pharmazeutische Technologie", Verfasser Prof. Bauer, Frömmig und Führer, Thieme Verlag, Seiten 292 ff., zu entnehmen, dass man Arzneiformen über Trockengranulierung aus der Schmelze gewinnen kann. Es wird beschrieben, dass Schmelzerstarrungsgranulate entweder durch Schmelzen und Schockerstarren, durch Ausgießen und Zerkleinern oder durch Sprüherstarren in Sprühtürmen hergestellt werden können. Ein Problem bei diesen Verfahren ist die für die Herstellung von Arzneimitteln erforderliche exakte Formgebung. Es werden häufig unregelmäßige Partikel oder Bruchstücke erzeugt, so dass die erzielte Form in keiner Weise den üblichen Arzneiformen entspricht und Granulate deshalb als eigenständige Arzneiform nur eine geringe Bedeutung besitzen. Die Herstellung der gewünschten festen Arzneiformen erfordert den Einsatz weiterer Verfahrensschritte, wie zum Beispiel die Komprimierung mit Hilfe von Tablettiermaschinen. Dies ist zeit- und kostenintensiv.

Seit einiger Zeit ist ein wesentlich einfacheres kontinuierliches Verfahren zur Herstellung fester pharmazeutischer Formen bekannt, bei dem man eine wirkstoffhaltige, lösungsmittelfreie Schmelze aus einem polymeren, wirkstoffhaltigen Bindemittel extrudiert und den extrudierten Strang zu der gewünschten Arzneiform formt, beispielsweise in einem Kalander mit Formwalzen, siehe EP-A-240 904, EP-A-240 906, EP-A-337 256, US-A-4,880,585 und EP-A-358105. Damit kann eine gezielte Formgebung erreicht werden. Als polymeres Bindemittel werden insbesondere Polymere des N-Vinylpyrrolidons oder Copolymerisate davon, z. B. mit Vinylacetat, eingesetzt.

EP 544144 beschreibt die Herstellung fester pharmazeutischer Dosierungsformen mit verzögerter WirkstoffFreisetzung (Retardformen). Diese werden durch Vermischen eines Wirkstoffs mit einer Polymerschmelze mittels Schmelzextrusion und anschließende Formgebung hergestellt. Die eingesetzte Polymerschmelze enthält wenigstens ein wasserunlösliches Poly(meth)acrylat und eine wasserlösliche Hydroxyalkylcellulose oder ein N-Vinylpyrrolidonpolymerisat.

Dosierungsformen auf Basis derartiger Polymere haben den Nachteil, dass sie den Wirkstoff relativ rasch freisetzen. Es ist daher nicht möglich, langsam freisetzende Dosierungsformen herzustellen, ohne zusätzliche Maßnahmen zu ergreifen, beispielsweise das Aufbringen eines die Freisetzung steuernden Überzugs.

Der vorliegenden Erfindung liegt daher die Aufgabe zugrunde, Dosierungsformen zur Verfügung zu stellen, die sich durch Schmelzextrusion herstellen lassen und in der Lage sind, den Wirkstoff langsam freizusetzen.

Überraschenderweise wurde nun gefunden, dass diese Aufgabe gelöst wird, wenn man als polymeres Bindemittel ein physiologisch verträgliches, wasserquellbares Pfropfcopolymerisat verwendet.

Gegenstand der vorliegenden Erfindung ist daher ein Verfahren zur Herstellung von festen Dosierungsformen durch Vermischen von mindestens einem polymeren Bindemittel, mindestens einem Wirkstoff und gegebenenfalls üblichen Additiven unter Bildung eines plastischen Gemisches und Formgebung, das dadurch gekennzeichnet ist, dass man als polymeres Bindemittel ein, insbesondere physiologisch verträgliches, wasserquellbares Pfropfcopolymerisat oder ein Gemisch von Pfropfcopolymerisaten einsetzt.

Das erfindungsgemäße Verfahren ermöglicht die Herstellung von festen Dosierungsformen mit sehr langsamer Freisetzung des Wirkstoffs ("sustained release", "slow release") auf einfache und kostengünstige Weise. Ein weiterer überraschender Vorteil des erfindungsgemäßen Verfahrens besteht darin, dass aufgrund der niedrigen Glasübergangstemperatur der einsetzbaren Pfropfcopolymerisate (< 90 °C) auch Formulierungen temperaturempfindlicher Wirkstoffe schonend zubereitet werden können. Darüberhinaus lässt sich die Freisetzungsrate durch Abmischungen mit rasch freisetzenden Polymeren wie Polyvinylpyrrolidon; Copolymerisaten von Polyvinylpyrrolidon und Vinylacetat; Celluloseethern, wie Hydroxypropylcellulose oder Hydroxyethylcellulose; Polyethylenglycolen oder Ethylenoxid/Propylenoxid-Blockcopolymeren (z. B. die Pluronic-Marken der BASF AG); Polyvinylalkoholen; teilverseiften Polyvinylalkoholen oder geeigneten niedermolekularen Substanzen, wie Zuckeralkoholen, Zuckern oder Salzen in einem weiten Bereich einstellen.

Unter Dosierungsformen sind hier alle Formen zu verstehen, die zur Verwendung als Arzneimittel, Pflanzenbehandlungsmittel, Futtermittel und Nahrungsmittel und zur Abgabe von Riechstoffen und Parfümölen geeignet sind. Dazu gehören beispielsweise Tabletten jeglicher Form, Pellets, Granulate, aber auch größere Formen, wie Würfel, Blöcke (Quader) oder zylindrische Formen, die sich insbesondere als Futter- oder Nahrungsmittel verwenden lassen.

Die erfindungsgemäß erhältlichen Dosierungsformen umfassen im Allgemeinen:
I 0 bis 90 Gew.-%, insbesondere 0,1 bis 60 Gew.-% (bezogen auf das Gesamtgewicht der Dosierungsform) eines Wirkstoffes,
II 10 bis 100 Gew.-%, insbesondere 40 bis 99,9 Gew.-% des polymeren Bindemittels und
III gegebenenfalls Additive.

Die erfindungsgemäß als Bindemittel verwendeten Polymere sind in an sich bekannter weise durch radikalische Polymerisation zugänglich. Die Herstellung erfolgt zum Beispiel mittels Lösungs-, Fällungs-, Suspensions- oder Emulsionspolymerisation unter Verwendung von Verbindungen, die unter den Polymerisationsbedingungen Radikale bilden. Vorzugsweise wird in dem erfindungsgemäßen Verfahren ein Pfropfcopolymerisat eingesetzt, das erhältlich ist durch radikalisch initiierte Polymerisation von
a) einer Komponente, die ausgewählt ist unter
   a1) C₁-C₃₀-Alkylestern einer α,β-monoethylenisch ungesättigten C₃-C₃₀-Mono- oder Dicarbonsäure. Bevorzugt sind die C₁-C₁₈-, insbesondere die C₁-C₈-Alkylester dieser Säuren. Von diesen Säuren sind die C₃-C₈-Mono- oder Dicarbonsäuren bevorzugt, wie Acrylsäure, Methacrylsäure, Dimethylacrylsäure, Ethacrylsäure, Maleinsäure, Citraconsäure, Methylenmalonsäure, Crotonsäure, Fumarsäure, Mesaconsäure und Itaconsäure , wobei Acrylsäure und Methacrylsäure besonders bevorzugt sind,
   a2) C₂-C₄-Hydroxyalkylestern der unter a1) erwähnten Säuren,
   a3) Amiden, Mono- oder Di-C₁-C₄-Alkylamiden und Nitrilen der unter a1) erwähnten Carbonsäuren,
b) sauerstoffhaltigen, vorzugsweise Alkylenoxideinheiten enthaltenden Polymerisaten als Pfropfgrundlage und
c) gegebenenfalls einem Monomer, das mindestens zwei ethylenisch ungesättigte, nicht-konjugierte Doppelbindungen aufweist.
wobei die Monomeren a) bis zu 5 Gew.-%, bezogen auf die Gesamtmenge der Monomeren a), einer α,β-monoethylenisch ungesättigten C₃-C₈-Carbonsäure, insbesondere Acrylsäure oder Methacrylsäure, umfassen können.

Zur Herstellung der Pfropfcopolymerisate sind als Monomere a) insbesondere Acrylsäuremethylester, Acrylsäureethylester, Methacrylsäuremethylester, Methacrylsäureethylester, Hydroxyethylacrylat, Hydroxypropylacrylat, Hydroxybutylacrylat, Hydroxyethylmethacrylat, Hydroxypropylmethacrylat, Hydroxyisobutylacrylat, Hydroxyisobutylmethacrylat, Maleinsäuremonomethylester, Maleinsäuredimethylester, Maleinsäuremonoethylester, Maleinsäurediethylester, 2-Ethylhexylacrylat, 2-Ethylhexylmethacrylat, Stearylacrylat, Stearylmethacrylat, Behenylacrylat, Behenylmethacrylat, Octylacrylat, Octylmethacrylat, Acrylamid, Methacrylamid, N-Dimethylacrylamid, N-tert.-butylacrylamid, Acrylnitril, Methacrylnitril oder Mischungen davon einsetzbar. Besonders bevorzugte Komponenten a) sind Acrylsäuremethylester, Acrylsäureethylester, Methacrylsäuremethylester, Methacrylsäureethylester, Acrylsäure-n-butylester, Acrylsäure-tert.-butylester oder Mischungen davon.

Die Pfropfgrundlagen sind vorzugsweise ausgewählt unter:
b1) Alkylenoxideinheiten enthaltenden Polymerisaten. Dazu zählen Homo- und Copolymerisate von C₂-C₄-Alkylenoxiden, Polytetrahydrofurane, die Umsetzungsprodukte von C₂-C₄-Alkylenoxiden mit C₁-C₃₀-Alkoholen, Fettsäuren, C₁-C₁₂-Alkylphenolen, primären oder sekundären, aliphatischen C₂-C₃₀-Aminen, oder Gemischen davon;
b2) Polyvinylalkoholen oder Copolymerisaten von Polyvinylalkohol (PVA) und Vinylacetat (VA)(teilverseiftes Polyvinylacetat), vorzugsweise im Gewichtsverhältnis PVA:VA = 95:5 bis 10:90;
b3) Stärke, Cellulose und Derivaten davon, wie Methylcellulose, Hydroxyethylcellulose, Hydroxypropylmethylcellulose und Carboxymethylcellulose.

Bei den Copolymerisaten von C₂-C₄-Alkylenoxiden kann es sich entweder um statistische Copolymerisate handeln, wenn man Mischungen aus mindestens 2 Alkylenoxiden polymerisiert, oder um Blockcopolymerisate, wenn man zunächst ein Alkylenoxid, beispielsweise Ethylenoxid, polymerisiert und dann ein anderes Alkylenoxid polymerisiert, z. B. Propylenoxid. Die Blockcopolymerisate können beispielsweise dem Typ AB, ABA oder BAB angehören, wobei A beispielsweise ein Polyethylenoxidblock und B ein Block aus Polypropylenoxid sein kann. Diese Copolymerisate können gegebenenfalls außerdem noch n-Butylenoxid und/oder Isobutylenoxid einpolymerisiert enthalten. Die Polyalkylenoxide enthalten mindestens drei Alkylenoxideinheiten im Molekül. Die Polyalkylenoxide können beispielsweise bis zu 50 000 Alkylenoxideinheiten im Molekül aufweisen. Die Polytetrahydrofurane enthalten beispielsweise 3 bis 200, vorzugsweise 3 bis 100 Tetramethylenoxideinheiten.

Besonders bevorzugt zum Einsatz gelangende Verbindungen b) sind neben den bereits genannten Homo- oder Blockcopolymerisaten von Ethylenoxid und Propylenoxid auch statistisch aufgebaute Ethylenoxid-Propylenoxid-Copolymerisate und Polyvinylalkohol.

Als Alkohole für die Umsetzungsprodukte von Alkylenoxiden mit Alkoholen (C₁- bis C₃₀-Alkohole) kommen beispielsweise aliphatische Monoalkohole, wie Methanol, Ethanol, n-Propanol, iso-Propanol, n-Butanol, n-Octanol, 2-Ethylhexanol, Decanol, Dodecanol, Palmitylalkohol, Cetylalkohol und Stearylalkohol in Betracht. Man kann aber auch zwei- und mehrwertige aliphatische Alkohole einsetzen, z. B. Glykol, Glycerin, Erythrit, Pentaerythrit und Sorbit. Die Alkohole werden im Molverhältnis 1:3 bis 1:200 mit mindestens einem C₂- bis C₄-Alkylenoxid umgesetzt.

Weitere geeignete Alkylenoxideinheiten enthaltende Polymere b1) sind Umsetzungsprodukte von Fettsäuren mit Alkylenoxiden. Von den Fettsäuren kommen insbesondere solche in Betracht, die 8 bis 10 C-Atome im Molekül enthalten, beispielsweise Laurinsäure, Myristinsäure, Stearinsäure, Palmitinsäure, Kokosfettsäure, Talgfettsäure und Ölsäure.

Alkylenoxideinheiten enthaltende Polymere b1) sind außerdem die Additionsprodukte von C₂- bis C₄-Alkylenoxiden an C₁- bis C₁₂-Alkylphenole, wie n-Cetylphenol, n-Octylphenol, Isobutylphenol und Methylphenol. Außerdem eignen sich als Komponente b) für die Herstellung der Pfropfpolymerisate die Anlagerungsprodukte von C₂bis C₄-Alkylenoxiden an primäre und sekundäre C₂- bis C₃₀-Amine, wie Di-n-butylamin, Di-n-octylamin, Dimethylamin und Distearylamin. Das Molverhältnis von Amin zu Alkylenoxid beträgt 1:3 bis 1:200 und liegt vorzugsweise in dem Bereich von 1:3 bis 1:100.

Für die Herstellung der Additionsprodukte von Alkylenoxiden an Alkohole, Phenole, Säuren oder Amine kann man die Alkylenoxide in bekannter Weise an die vorstehend genannten Verbindungen gleichzeitig und nacheinander addieren. Bei der nacheinander erfolgenden Addition der Alkylenoxide entstehen Blockcopolymerisate. In manchen Fällen kann es außerdem noch von Vorteil sein, die freien OH-Gruppen der Alkoxylierungsprodukte mit einer Endgruppe zu verschließen. Der Endgruppenverschluss kann beispielsweise mit einem Alkylrest unter Ausbildung einer Ethergruppe erfolgen. Beispielsweise kann man die Alkoxylierungsprodukte mit Alkylierungsmitteln, wie Dimethylsulfat umsetzen. Die endständigen OH-Gruppen können gegebenenfalls auch durch Umsetzung mit Carbonsäuren, z. B. Essigsäure oder Stearinsäure, verestert werden.

Eine Modifizierung der Pfropfcopolymerisate kann dadurch erreicht werden, dass man die Monomere oder Monomermischungen der Komponente a) mit bis zu 5 Gew.-%, vorzugsweise 0,05 bis 2 Gew.-%, eines oder mehrerer, mindestens zwei ethylenisch ungesättigte, nicht-konjugierte Doppelbindungen im Molekül aufweisenden Monomeren der Komponente c) in Gegenwart der Komponente b) copolymerisiert. Die Komponenten c) werden üblicherweise bei Copolymerisationen als Vernetzer verwendet. Geeignete Komponenten c) sind beispielsweise Methylenbisacrylamid, Divinylethylenharnstoff, Ester von Acrylsäure und Methacrylsäure mit mehrwertigen Alkoholen, wie Glykoldiacrylat, Glycerintriacrylat, Glykoldimethacrylat, Glycerintrimethacrylat, sowie mindestens zweifach mit Acrylsäure oder Methacrylsäure veresterte Polyole, wie Pentaerythrit und Glucose. Geeignete als Komponente c) einsetzbare Vernetzer sind außerdem Divinylbenzol, Divinyldioxan, Pentaerythrittriallylether und Pentaallylsucrose.

Vorzugsweise setzt man die Komponenten a) und b) in Gewichtsverhältnis im Bereich von 95 bis 10 : 5 bis 90, insbesondere 85 bis 55 : 15 bis 45 ein.

Die Polymerisationstemperaturen bei der Herstellung der erfindungsgemäß einsetzbaren Pfropfcopolymerisate liegen üblicherweise im Bereich von 30 bis 200 °C, vorzugsweise 40 bis 110 °C. Geeignete Initiatoren sind beispielsweise übliche Azo- und Peroxyverbindungen sowie die üblichen Redoxinitiatorsysteme, wie Kombinationen aus Wasserstoffperoxid und reduzierend wirkenden Verbindungen, beispielsweise Natriumsulfit, Natriumbisulfit, Natriumformaldehydsulfoxylat und Hydrazin.

Die Pfropfpolymerisation kann auch durch Einwirkung von ultravioletter Strahlung, gegebenenfalls in Gegenwart von UV-Initiatoren, durchgeführt werden. Für die Polymerisation unter Einwirkung von UV-Strahlen setzt man die dafür üblicherweise in Betracht kommenden Photoinitiatoren bzw. Sensibilisatoren ein, wie Benzoin und Benzoinether, α-Methylbenzoin oder α-Phenylbenzoin. Auch sog. Triplett-Sensibilisatoren, wie Benzildiketale, können verwendet werden. Als UV-Strahlungsquellen dienen beispielsweise neben energiereichen UV-Lampen, wie Kohlenbogenlampen, Quecksilberdampflampen oder Xenonlampen, auch UV-arme Lichtquellen, wie Leuchtstoffröhren mit hohem Blauanteil.

Zur Erzielung niedriger Molekulargewichte können die üblichen Regler eingesetzt werden, beispielsweise Verbindungen, die Schwefel in gebundener Form enthalten, wie Alkylmercaptane.

Die Homo- und Copolymerisate besitzen im Allgemeinen K-Werte von mindestens 7, vorzugsweise 10 bis 100, insbesondere 20 bis 100, besonders bevorzugt 20 bis 35. Die Bestimmung der K-Werte erfolgt nach H. Fikentscher, Cellulosechemie, Band 13, 58-64 und 71-74 (1932), in wässriger Lösung oder in einem organischen Lösungsmittel bei 25 °C und bei Konzentrationen, die je nach K-Wert-Bereich zwischen 0,1 % und 5 % liegen.

Neben den oben beschriebenen, erfindungsgemäß einsetzbaren polymeren Bindemitteln können, insbesondere bis zu 30 Gew.-%, bezogen auf das Gesamtgewicht des Bindemittels, weitere Bindemittel eingesetzt werden, wie Polymere, Copolymere, Cellulosederivate, Stärke und Stärkederivate. Geeignet sind beispielsweise:

Polyvinylpyrrolidon (PVP), Copolymerisate von N-Vinylpyrrolidon (NVP) und Vinylestern, insbesondere Vinylacetat, Copolymerisate von Vinylacetat und Crotonsäure, teilverseiftes Polyvinylacetat, Polyvinylalkohol, Polyhydroxyalkylacrylate, Polyhydroxyalkylmethacrylate, Polyacrylate und Polymethacrylate (Eudragit-Typen), Copolymerisate von Methylmethacrylat und Acrylsäure, Polyacrylamide, Polyethylenglykole, Celluloseester, Celluloseether, insbesondere Methylcellulose und Ethylcellulose, Hydroxyalkylcellulosen, insbesondere Hydroxypropylcellulose, Hydroxyalkyl-Alkylcellulosen, insbesondere Hydroxypropyl-Ethylcellulose, Cellulosephthalate, insbesondere Celluloseacetatphthalat und Hydroxypropylmethylcellulosephthalat, und Mannane, insbesondere Galactomannane. Davon sind Polyvinylpyrrolidon, Copolymerisate von N-Vinylpyrrolidon und Vinylestern, Polyhydroxyalkylacrylate, Polyhydroxyalkylmethacrylate, Polyacrylate, Polymethacrylate, Alkylcellulosen und Hydroxyalkylcellulosen besonders bevorzugt.

Das erfindungsgemäß einsetzbare polymere Bindemittel muß in der Gesamtmischung aller Komponenten im Bereich von 50 bis 180 °C, vorzugsweise 60 bis 130 °C erweichen oder schmelzen. Die Glasübergangstemperatur der Mischung muss daher unter 180 °C, vorzugsweise unter 130 °C, insbesondere unter 90 °C liegen. Erforderlichenfalls wird sie durch übliche, pharmakologisch akzeptable weichmachende Hilfsstoffe herabgesetzt. Die Menge an Weichmacher beträgt höchstens 30 Gew.-%, bezogen auf das Gesamtgewicht von Bindemittel und Weichmacher, damit lagerstabile Arzneiformen gebildet werden, die keinen kalten Fluss zeigen. Vorzugsweise enthält das Gemisch aber keinen Weichmacher.

Beispiele für derartige Weichmacher sind:
langkettige Alkohole, Ethylenglykol, Propylenglykol, Glycerin, Trimethylolpropan, Triethylenglykol, Butandiole, Pentanole, wie Pentaerythrit, Hexanole, Polyethylenglykole, Polypropylenglykole, Polyethylen-propylenglykole, Silicone, aromatische Carbonsäureester (z. B. Dialkylphthalate, Trimellithsäureester, Benzoesäureester, Terephthalsäureester) oder aliphatische Dicarbonsäureester (z. B. Dialkyladipate, Sebacinsäureester, Azelainsäureester, Zitronen- und Weinsäureester), Fettsäureester, wie Glycerinmono-, Glycerindi- oder Glycerintriacetat oder Natriumdiethylsulfosuccinat. Die Konzentration an Weichmacher beträgt im Allgemeinen 0,5 bis 15, vorzugsweise 0,5 bis 5 Gew.-%, bezogen auf das Gesamtgewicht des Gemisches.

Übliche galenische Hilfsstoffe, deren Gesamtmenge bis zu 100 Gew.-%, bezogen auf das Polymerisat, betragen kann, sind z. B. Streckmittel bzw. Füllstoffe, wie Silikate oder Kieselerde, Magnesiumoxid, Aluminiumoxid, Titanoxid, Stearinsäure oder deren Salze, z. B. das Magnesium- oder Calciumsalz, Methylcellulose, Natrium-Carboxymethylcellulose, Talkum, Saccharose, Lactose, Getreide- oder Maisstärke, Kartoffelmehl, Polyvinylalkohol, insbesondere in einer Konzentration von 0,02 bis 50, vorzugsweise 0,20 bis 20 Gew.-%, bezogen auf das Gesamtgewicht des Gemisches.

Schmiermittel, wie Aluminium- und Calciumstearat, Talkum und Silicone, in einer Konzentration von 0,1 bis 5, vorzugsweise 0,1 bis 3 Gew.-%, bezogen auf das Gesamtgewicht des Gemisches.

Fließmittel, wie tierische oder pflanzliche Fette, insbesondere in hydrierter Form und solche, die bei Raumtemperatur fest sind. Diese Fette haben vorzugsweise einen Schmelzpunkt von 50°C oder höher. Bevorzugt sind Triglyceride der C₁₂-, C₁₄-, C₁₆- und C₁₈-Fettsäuren. Auch Wachse, wie Carnaubawachs, sind brauchbar. Diese Fette und Wachse können vorteilhaft alleine oder zusammen mit Mono- und/oder Diglyceriden oder Phosphatiden, insbesondere Lecithin, zugemischt werden. Die Mono- und Diglyceride stammen vorzugsweise von den oben erwähnten Fettsäuretypen ab. Die Gesamtmenge an Fetten, Wachsen, Mono-, Diglyceriden und/oder Lecithinen beträgt 0,1 bis 30, vorzugsweise 0,1 bis 5 Gew.-%, bezogen auf das Gesamtgewicht der Masse für die jeweilige Schicht;

Farbstoffe, wie Azofarbstoffe, organische oder anorganische Pigmente oder Farbstoffe natürlicher Herkunft, wobei anorganische Pigmente in einer Konzentration von 0,001 bis 10, vorzugsweise 0,5 bis 3 Gew.-%, bezogen auf das Gesamtgewicht des Gemisches bevorzugt sind;

Stabilisatoren, wie Antioxidanzien, Lichtstabilisatoren, Hydroperoxid-Vernichter, Radikalfänger, Stabilisatoren gegen mikrobiellen Befall.

Ferner können Netz-, Konservierungs-, Spreng-, Adsorptions-, Formentrenn- und Treibmittel zugesetzt werden (vgl. z. B. H. Sucker et al., Pharmazeutische Technologie, Thieme-Verlag, Stuttgart 1978).

Unter Hilfsstoffen im Sinne der Erfindung sind auch Substanzen zur Herstellung einer festen Lösung des Wirkstoffs zu verstehen. Diese Hilfsstoffe sind beispielsweise Pentaerythrit und Pentaerythrit-tetraacetat, Polymere wie z. B. Polyethylen- bzw. Polypropylenoxide und deren Blockcopolymere (Poloxamere), Phosphatide wie Lecithin, Homo- und Copolymere des Vinylpyrrolidons, Tenside wie Polyoxyethylen-40-stearat sowie Zitronen- und Bernsteinsäure, Gallensäuren, Sterine und andere wie z. B. bei J. L. Ford, Pharm. Acta Helv. 61, 69-88 (1986) angegeben.

Als Hilfsstoffe gelten auch Zusätze von Basen und Säuren zur Steuerung der Löslichkeit eines Wirkstoffes (siehe beispielsweise K. Thoma et al., Pharm. Ind. 51, 98-101 (1989)).

Einzige Voraussetzung für die Eignung von Hilfsstoffen ist eine ausreichende Temperaturstabilität.

Unter Wirkstoffen im Sinne der Erfindung sind alle Stoffe mit einer physiologischen Wirkung zu verstehen, sofern sie sich unter den Verarbeitungsbedingungen nicht zersetzen. Es handelt sich insbesondere um pharmazeutische Wirkstoffe (für Mensch und Tier), Wirkstoffe für die Pflanzenbehandlung, Insektizide, Futter- und Nahrungsmittelwirkstoffe, Riechstoffe und Parfümöle. Die Wirkstoffmenge pro Dosiseinheit und die Konzentration können je nach Wirksamkeit und Freisetzungsgeschwindigkeit in weiten Grenzen variieren. Die einzige Bedingung ist, dass sie zur Erzielung der gewünschten Wirkung ausreichen. So kann die Wirkstoffkonzentration im Bereich von 0,1 bis 95, vorzugsweise von 20 bis 80, insbesondere 30 bis 70 Gew.-% liegen. Auch Wirkstoff-Kombinationen können eingesetzt werden. Wirkstoffe im Sinne der Erfindung sind auch Vitamine und Mineralstoffe. Zu den Vitaminen gehören die Vitamine der A-Gruppe, der B-Gruppe, worunter neben B₁, B₂, B₆ und B₁₂ sowie Nicotinsäure und Nicotinamid auch Verbindungen mit Vitamin B-Eigenschaften verstanden werden, wie z. B. Adenin, Cholin, Pantothensäure, Biotin, Adenylsäure, Folsäure, Orotsäure, Pangamsäure, Carnitin, p-Aminobenzoesäure, myo-Inosit und Liponsäure sowie Vitamin C, Vitamine der D-Gruppe, E-Gruppe, F-Gruppe, H-Gruppe, I- und J-Gruppe, K-Gruppe und P-Gruppe. Zu Wirkstoffen im Sinne der Erfindung gehören auch Peptidtherapeutika. Zu Pflanzenbehandlungsmitteln zählen z. B. Vinclozolin, Epoxiconazol und Quinmerac.

Das erfindungsgemäße Verfahren ist beispielsweise zur Verarbeitung folgender Wirkstoffe geeignet:
Acebutolol, Acetylcystein, Acetylsalicylsäure, Acyclovir, Alfacalcidol, Allantoin, Allopurinol, Alprazolam, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benserazid, Benzalkonium-Hydrochlorid, Benzocain, Benzoesäure, Betamethason, Bezafibrat, Biotin, Biperiden, Bisoprolol, Bromazepam, Bromhexin, Bromocriptin, Budesonid, Bufexamac, Buflomedil, Buspiron, Coffein, Campher, Captopril, Carbamazepin, Carbidopa, Carboplatin, Cefachlor, Cefalexin, Cefadroxil, Cefazolin, Cefixim, Cefotaxim, Ceftazidim, Ceftriaxon, Cefuroxim, Chloramphenicol, Chlorhexidin, Chlor-pheniramin, Chlortalidon, Cholin, Cyclosporin, Cilastatin, Cimetidin, Ciprofloxacin, Cisapride, Cisplatin, Clarithromycin, Clävulansäure, Clomipramin, Clonazepam, Clonidin, Clotrimazol, Codein, Cholestyramin, Cromoglycinsäure, Cyanocobalamin, Cyproteron, Desogestrel, Dexamethason, Dexpanthenol, Dextromethorphan, Dextropropoxiphen, Diazepam, Diclofenac, Digoxin, Dihydrocodein, Dihydroergotamin, Dihydroergotoxin, Diltiazem, Diphenhydramin, Dipyridamol, Dipyron, Disopyramid, Domperidon, Dopamin, Doxycyclin, Enalapril, Ephedrin, Epinephrin, Ergocalciferol, Ergotamin, Erythromycin, Estradiol, Ethinylestradiol, Etoposid, Eucalyptus Globulus, Famotidin, Felodipin, Fenofibrat, Fenoterol, Fentanyl, Flavin-Mononucleotid, Fluconazol, Flunarizin, Fluorouracil, Fluoxetin, Flurbiprofen, Folinsäure, Furosemid, Gallopamil, Gemfibrozil, Gentamicin, Gingko Biloba, Glibenclamid, Glipizid, Clozapin, Glycyrrhiza glabra, Griseofulvin, Guaifenesin, Haloperidol, Heparin, Hyaluronsäure, Hydrochlorothiazid, Hydrocodon, Hydrocortison, Hydromorphon, Ipratropium-Hydroxid, Ibuprofen, Imipenem, Imipramin, Indomethacin, Iohexol, Iopamidol, Isosorbid-Dinitrat, Isosorbid-Mononitrat, Isotretinoin, Itraconazol, Ketotifen, Ketoconazol, Ketoprofen, Ketorolac, Labetalol, Lactulose, Lecithin, Levocarnitin, Levodopa, Levoglutamid, Levonorgestrel, Levothyroxin, Lidocain, Lipase, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamin-Mischungen bzw. -Kombinationen und Mineralsalze, N-Methylephedrin, Naftidrofuryl, Naproxen, Neomycin, Nicardipin, Nicergolin, Nicotinamid, Nicotin, Nicotinsäure, Nifedipin, Nimodipin, Nitrazepam, Nitrendipin, Nizatidin, Norethisteron, Norfloxacin, Norgestrel, Nortriptylin, Nystatin, Ofloxacin, Omeprazol, Ondansetron, Pancreatin, Panthenol, Pantothensäure, Paracetamol, Penicillin G, Penicillin V, Phenobarbital, Pentoxifyllin, Phenoxymethylpenicillin, Phenylephrin, Phenylpropanolamin, Phenytoin, Piroxicam, Polymyxin B, Povidon-Iod, Pravastatin, Prazepam, Prazosin, Prednisolon, Prednison, Propafenon, Propranolol, Proxyphyllin, Pseudoephedrin, Pyridoxin, Quinidin, Ramipril, Ranitidin, Reserpin, Retinol, Riboflavin, Rifampicin, Rutosid, Saccharin, Salbutamol, Salcatonin, Salicylsäure, Selegilin, Simvastatin, Somatropin, Sotalol, Spironolacton, Sucralfat, Sulbactam, Sulfamethoxazol, Sulfasalazin, Sulpirid, Tamoxifen, Tegafur, Teprenon, Terazosin, Terbutalin, Terfenadin, Tetracyclin, Theophyllin, Thiamin, Ticlopidin, Timolol, Tranexamsäure, Tretinoin, Triamcinolon-Acetonid, Triamteren, Trimethoprim, Troxerutin, Uracil, Valproinsäure, Vancomycin, Verapamil, Vitamin E, Zidovudin.

Bevorzugte Wirkstoffe sind Ibuprofen (als Racemat, Enantiomer oder angereichertes Enantiomer), Ketoprofen, Flurbiprofen, Acetylsalicylsäure, Verapamil, Paracetamol, Nifedipin oder Captopril.

Zur Herstellung der festen Dosierungsformen wird ein plastisches Gemisch der Komponenten (Schmelze) bereitgestellt, das anschließend einem Formgebungsschritt unterzogen wird. Das Vermischen der Komponenten und die Bildung der Schmelze können auf unterschiedliche Weise erfolgen. Das Vermischen kann vor, während und/oder nach der Bildung der Schmelze erfolgen. Beispielsweise können die Komponenten zuerst vermischt und dann aufgeschmolzen oder gleichzeitig vermischt und aufgeschmolzen werden. Häufig erfolgt noch eine Homogenisierung des plastischen Gemisches, um eine hochdisperse Verteilung des Wirkstoffes zu erhalten.

Insbesondere bei Verwendung von empfindlichen Wirkstoffen hat es sich aber als bevorzugt erwiesen, zuerst das polymere Bindemittel, gegebenenfalls zusammen mit üblichen pharmazeutischen Additiven, aufzuschmelzen und vorzuvermischen und dann den (die) empfindlichen Wirkstoff(e) in "Intensivmischern" in plastischer Phase bei sehr kleinen Verweilzeiten einzumischen (Homogenisieren). Der (die) Wirkstoff(e) kann (können) dabei in fester Form oder als Lösung oder Dispersion eingesetzt werden.

Im Allgemeinen werden die Komponenten als solche in das Herstellungsverfahren eingesetzt. Sie können jedoch auch in flüssiger Form, d. h. als Lösung, Suspension oder Dispersion zur Anwendung kommen.

Als Lösungsmittel für die flüssige Form der Komponenten kommt in erster Linie Wasser oder ein mit Wasser mischbares, organisches Lösungsmittel oder ein Gemisch davon mit Wasser in Betracht. Brauchbare Lösungsmittel sind aber auch mit Wasser nicht mischbare oder mischbare, organische Lösungsmittel. Geeignete, mit Wasser mischbare Lösungsmittel sind insbesondere C₁-C₄-Alkanole, wie Ethanol, Isopropanol oder n-Propanol, Polyole, wie Ethylenglykol, Glycerin und Polyethylenglykole. Geeignete, mit Wasser nicht mischbare Lösungsmittel sind Alkane, wie Pentan oder Hexan, Ester, wie Ethylacetat oder Butylacetat, chlorierte Kohlenwasserstoffe, wie Methylenchlorid und aromatische Kohlenwasserstoffe, wie Toluol und Xylol. Ein weiteres brauchbares Lösungsmittel ist flüssiges CO₂.

Welches Lösungsmittel im Einzelfall verwendet wird, hängt von der aufzunehmenden Komponente und deren Eigenschaften ab. Beispielsweise kommen pharmazeutische Wirkstoffe häufig in Form eines Salzes, das im Allgemeinen wasserlöslich ist, zur Anwendung. Wasserlösliche Wirkstoffe können daher als wässrige Lösung eingesetzt werden oder vorzugsweise in die wässrige Lösung oder Dispersion des Bindemittels aufgenommen werden. Entsprechendes gilt für Wirkstoffe, die in einem der genannten Lösungsmittel löslich sind, wenn die flüssige Form der zur Anwendung kommenden Komponenten auf einem organischen Lösungsmittel basiert.

Gegebenenfalls kann an die Stelle des Aufschmelzens ein Lösen, Suspendieren oder Dispergieren in den oben genannten Lösungsmitteln, falls erwünscht und/oder erforderlich unter Zusatz geeigneter Hilfsstoffe, wie z. B. Emulgatoren, treten. Das Lösungsmittel wird dann im Allgemeinen unter Bildung der Schmelze in einer geeigneten Apparatur, z. B. einem Extruder, entfernt. Im Folgenden soll dies von dem Begriff Vermischen umfasst werden.

Das Aufschmelzen und/oder Vermischen erfolgt in einer für diesen Zweck üblichen Vorrichtung. Besonders geeignet sind Extruder oder gegebenenfalls beheizbare Behälter mit Rührwerk, z. B. Kneter, (wie der unten noch erwähnten Art).

Als Mischapparat sind insbesondere solche Vorrichtungen brauchbar, die in der Kunststofftechnologie zum Mischen eingesetzt werden. Geeignete Vorrichtungen sind beispielsweise beschrieben in "Mischen beim Herstellen und Verarbeiten von Kunststoffen", H. Pahl, VDI-Verlag, 1986. Besonders geeignete Mischapparaturen sind Extruder und dynamische und statische Mischer, sowie Rührkessel, einwellige Rührwerke mit Abstreifvorrichtungen, insbesondere sogenannte Pastenrührwerke, mehrwellige Rührwerke, insbesondere PDSM-Mischer, Feststoffmischer sowie vorzugsweise Misch-Knetreaktoren (z. B. ORP, CRP, AP, DTB der Firma List oder Reactotherm der Firma Krauss-Maffei oder Ko-Kneter der Fa. Buss), Doppelmuldenkneter (Trogmischer) und Stempelkneter (Innenmischer) oder Rotor/Stator-Systeme (z. B. Dispax der Firma IKA).

Bei empfindlichen Wirkstoffen erfolgt vorzugsweise zunächst das Aufschmelzen des polymeren Bindemittels in einem Extruder und anschließend das Zumischen des Wirkstoffs in einem Misch-Knetreaktor. Bei weniger empfindlichen Wirkstoffen kann man dagegen zum intensiven Dispergieren des Wirkstoffs ein Rotor/Stator-System einsetzen.

Das Beschicken der Mischvorrichtung erfolgt je nach deren Konzeption kontinuierlich oder diskontinuierlich in üblicher Weise. Pulverförmige Komponenten können im freien Zulauf, z. B. über eine Differentialdosierwaage eingeführt werden. Plastische Massen können direkt aus einem Extruder eingespeist oder über eine Zahnradpumpe, die insbesondere bei hohen Viskositäten und hohen Drüken von Vorteil ist, zugespeist werden. Flüssige Medien können über ein geeignetes Pumpenaggregat zudosiert werden.

Das durch Vermischen und/oder Aufschmelzen des Bindemittels, des Wirkstoffes und gegebenenfalls des Additivs oder der Additive erhaltene Gemisch ist teigig bis zähflüssig (thermoplastisch) oder flüssig und daher extrudierbar. Die Glasübergangstemperatur des Gemisches liegt unter der Zersetzungstemperatur aller in dem Gemisch enthaltenen Komponenten. Das Bindemittel soll vorzugsweise in physiologischer Umgebung löslich oder quellbar sein.

Die Verfahrensschritte Vermischen und Aufschmelzen können in derselben Apparatur oder in zwei oder mehreren getrennt arbeitenden Vorrichtungen ausgeführt werden. Die Zubereitung einer Vormischung kann in einer der oben beschriebenen üblichen Mischvorrichtungen durchgeführt werden. Eine solche Vormischung kann dann direkt, z. B. in einen Extruder, eingespeist und anschließend ggf. unter Zusatz weiterer Komponenten extrudiert werden.

Das erfindungsgemäße Verfahren erlaubt es, als Extruder Einschneckenmaschinen, kämmende Schneckenmaschinen oder auch Mehrwellextruder, insbesondere Zweischnecken-Extruder, gleichsinnig oder gegensinnig drehend und gegebenenfalls mit Knetscheiben ausgerüstet, einzusetzen. Wenn bei der Extrusion ein Lösungsmittel verdampft werden muss, sind die Extruder im Allgemeinen mit einem Verdampfungsteil ausgerüstet. Besonders bevorzugt sind Extruder der ZSK-Baureihe von Werner u. Pfleiderer.

Erfindungsgemäß können auch mehrschichtige pharmazeutische Formen durch Koextrusion hergestellt werden, wobei mehrere Gemische aus den oben beschriebenen Komponenten bei der Extrusion so in einem Werkzeug zusammengeführt werden, dass sich der gewünschte Schichtaufbau der mehrschichtigen pharmazeutischen Form ergibt. Vorzugsweise verwendet man verschiedene Bindemittel für verschiedene Schichten.

Mehrschichtige Arzneiformen umfassen vorzugsweise zwei oder drei Schichten. Sie können in offener oder geschlossener Form vorliegen, insbesondere als offene oder geschlossene Mehrschichttabletten.

Wenigstens eine der Schichten enthält wenigstens einen pharmazeutischen Wirkstoff. Es ist auch möglich, einen weiteren Wirkstoff in eine andere Schicht aufzunehmen. Dies hat den Vorteil, dass zwei miteinander unverträgliche Wirkstoffe verarbeitet werden können oder dass die Freisetzungscharakteristik des Wirkstoffes gesteuert werden kann.

Das Ausformen erfolgt durch Koextrusion, wobei die Gemische aus den einzelnen Extrudern oder anderen Aggregaten in ein gemeinsames Koextrusionswerkzeug geführt und ausgetragen werden. Die Form der Koextrusionswerkzeuge richtet sich nach der gewünschten pharmazeutischen Form. Beispielsweise sind Werkzeuge mit ebenem Austrittsspalt, sogenannte Breitschlitzwerkzeuge, und Werkzeuge mit kreisringspaltförmigem Austrittsquerschnitt geeignet. Die Düsenauslegung erfolgt dabei in Abhängigkeit von dem zur Anwendung kommenden polymeren Bindemittel und der gewünschten pharmazeutischen Form.

Das erhaltene Gemisch ist vorzugsweise lösungsmittelfrei, d.h. es enthält weder Wasser noch ein organisches Lösungsmittel.

Das plastische Gemisch wird in der Regel einer abschließenden Formgebung unterzogen. Dabei kann eine Vielzahl von Formen, je nach Werkzeug und Art der Formung, erzeugt werden. Beispielsweise lässt sich bei Verwendung eines Extruders der extrudierte Strang zwischen einem Band und einer Walze, zwischen zwei Bändern oder zwischen zwei Walzen, wie in der EP-A-358 105 beschrieben, oder durch Kalandrierung in einem Kalander mit zwei Formwalzen, siehe beispielsweise EP-A-240 904, formen. Durch Extrusion und Heißoder Kaltabschlag des Stranges können weitere Formen erhalten werden, beispielsweise kleinteilige und gleichmäßig geformte Granulate. Die Heißgranulierung führt in der Regel zu linsenförmigen Dosierungsformen (Tabletten) mit einem Durchmesser von 1 bis 10 mm, während die Kaltgranulierung normalerweise zu zylinderförmigen Produkten mit einem Verhältnis von Länge zu Durchmesser von 1 bis 10 und einem Durchmesser von 0,5 bis 10 mm führt. So können einschichtige, bei Anwendung der Koextrusion aber auch offene oder geschlossene, mehrschichtige Dosierungsformen hergestellt werden, beispielsweise Oblongtabletten, Dragees, Pastillen und Pellets. Die erhaltenen Granulate können anschließend auch zu Pulver gemahlen und in üblicher Weise zu Tabletten verpresst werden. Mikropastillen können durch das Rotoform-Sandvik-Verfahren hergestellt werden. Diese Dosierungsformen können in einem nachgeschalteten Verfahrensschritt nach üblichen Methoden gerundet und/oder mit einem Coating versehen werden. Geeignete Materialien für Filmüberzüge sind z. B. Polyacrylate, wie die Eudragit-Typen, Celluloseester, wie die Hydroxypropylcellulosephthalate, sowie Celluloseether, wie Ethylcellulose, Hydroxypropylmethylcellulose oder Hydroxypropylcellulose.

Im Einzelnen kann es zur Ausbildung von festen Lösungen kommen. Der Begriff "feste Lösungen" ist dem Fachmann geläufig, beispielsweise aus der eingangs zitierten Literatur. In festen Lösungen von Wirkstoffen in Polymeren liegt der Wirkstoff molekulardispers im Polymer vor.

Die folgenden Beispiele sollen das erfindungsgemäße Verfahren veranschaulichen, ohne es jedoch zu beschränken.

### Beispiele

### Beispiel 1: Synthese eines Pfropfcopolymers aus 40 Gew.-% eines sauerstoffhaltigen Blockcopolymers (Pfropfgrundlage) und 60 Gew.-% Methacrylsäuremethylester (aufgepfropftes Monomer)

Eine Mischung aus 240 g eines ABA-Blockcopolymers (Poloxamer 188; A = Polyethylenoxidblock, B = Polypropylenoxidblock) und 440 g n-Propanol wurde unter Rühren in einer Stickstoffatmosphäre auf 92 °C aufgeheizt. Nach Erreichen der Innentemperatur von 92 °C erfolgte die Zugabe von 70 g Methacrylsäuremethylester und 21 g einer Mischung aus 3 g tert.-Butylperoctoat und 120 g n-Propanol. Anschließend wurden bei 92 °C und ständigem Rühren 290 g Methacrylsäuremethylester in 2 Stunden und die restliche Mischung aus 3 g tert.-Butylperoctoat und 120 g n-Propanol in 2,5 Stunden zudosiert. Nach Beendigung des Initiatorzulaufs wurde zwei Stunden unter Rückfluss zum Sieden erhitzt und nach Zugabe von weiteren 0,6 g tert.-Butylperoctoat 3 Stunden unter Rückfluss nachpolymerisiert. Es wurden 600 g Wasser zugegeben und die Reaktionsmischung wurde über Nacht abgekühlt. Am nächsten Morgen wurde das n-Propanol gegen Wasser ausgetauscht (Wasserdampfdestillation) und das ausgefallene Produkt abfiltriert (Nutsche) und mit reichlich Wasser gewaschen. Es wurde ein weißes Pulver erhalten. K-Wert (1%ig in Aceton): 22,9; DSC: 1 Glasübergangsstufe bei 58 °C (2. Aufheizzyklus).

### Beispiel 2: Synthese eines Pfropfcopolymers aus 30 Gew.-% eines sauerstoffhaltigen Blockcopolymers (Pfropfgrundlage) und 70 Gew.-% Methacrylsäuremethylester (aufgepfropftes Monomer)

Eine Mischung aus 180 g des ABA-Blockcopolymers (Poloxamer 188; A = Polyethylenoxidblock, B = Polypropylenoxidblock) und 440 g n-Propanol wurde unter Rühren in einer Stickstoffatmosphäre auf 92 °C aufgeheizt. Nach Erreichen der Innentemperatur von 92 °C erfolgte die Zugabe von 70 g Methacrylsäuremethylester und 21 g einer Mischung aus 3 g tert.-Butylperoctoat und 120 g n-Propanol. Anschließend wurden bei 92 °C und ständigem Rühren 350 g Methacrylsäuremethylester in 2 Stunden und die restliche Mischung aus 3 g tert.-Butylperoctoat und 120 g n-Propanol in 2,5 Stunden zudosiert. Nach Beendigung des Initiatorzulaufs wurde zwei Stunden unter Rückfluss zum Sieden erhitzt und nach Zugabe von weiteren 0,6 g tert.-Butylperoctoat 3 Stunden unter Rückfluss nachpolymerisiert. Es wurden 600 g Wasser zugegeben und die Reaktionsmischung wurde über Nacht abgekühlt. Am nächsten Morgen wurde das n-Propanol gegen Wasser ausgetauscht (Wasserdampfdestillation) und das ausgefallene Produkt abfiltriert (Nutsche) und mit reichlich Wasser gewaschen. Es wurde ein weißes Pulver erhalten. K-Wert (1%ig in Aceton): 29,1; DSC: 1 Glasübergangsstufe bei 67 °C (2. Aufheizzyklus).

### Beispiel 3: Synthese eines Pfropfcopolymers aus 20 Gew.-% eines sauerstoffhaltigen Blockcopolymers (Pfropfgrundlage) und 80 Gew.-% Methacrylsäuremethylester (aufgepfropftes Monomer)

Eine Mischung aus 120 g des ABA-Blockcopolymers aus Beispiel 1 (A = Polyethylenoxidblock, B = Polypropylenoxidblock) und 440 g n-Propanol wurde unter Rühren in einer Stickstoffatmosphäre auf 92 °C aufgeheizt. Nach Erreichen der Innentemperatur von 92 °C erfolgte die Zugabe von 70 g Methacrylsäuremethylester und 21 g einer Mischung aus 3 g tert.-Butylperoctoat und 120 g n-Propanol.

Anschließend wurden bei 92 °C und ständigem Rühren 410 g Methacrylsäuremethylester in 2 Stunden und die restliche Mischung aus 3 g tert.-Butylperoctoat und 120 g n-Propanol in 2,5 Stunden zudosiert. Nach Beendigung des Initiatorzulaufs wurde zwei Stunden unter Rückfluss zum Sieden erhitzt und nach Zugabe von weiteren 0,6 g tert.-Butylperoctoat 3 Stunden unter Rückfluss nachpolymerisiert. Es wurden 600 g Wasser zugegeben und die Reaktionsmischung wurde über Nacht abgekühlt. Am nächsten Morgen wurde das n-Propanol gegen Wasser ausgetauscht (Wasserdampfdestillation) und das ausgefallene Produkt abfiltriert (Nutsche) und mit reichlich Wasser gewaschen. Es wurde ein weißes Pulver erhalten. K-Wert (1%ig in Aceton): 30,2; DSC: 1 Glasübergangsstufe bei 78 °C (2. Aufheizzyklus).

### Beispiel 4: 520 g des Pfropfcopolymers aus Beispiel 1 wurden mit 480 g Verapamil-Hydrochlorid unter folgenden Bedingungen extrudiert und zu 1000 mg-Oblong-Tabletten kalandriert.

| | |
|---|---|
| Schuss 1 | 67 °C |
| Schuss 2 | 98 °C |
| Schuss 3 | 131 °C |
| Schuss 4 | 111 °C |
| Schuss 5 | 97 °C |
| Düse | 81 °C |

Die Freisetzung nach 8 Stunden betrug 40 % [Paddlemodell nach USP (pH change)].

### Beispiel 5: 520 g des Pfropfcopolymers aus Beispiel 2 wurden mit 480 g Verapamil-Hydrochlorid unter folgenden Bedingungen extrudiert und zu 1000 mg-Oblong-Tabletten kalandriert.

| | |
|---|---|
| Schuss 1 | 63 °C |
| Schuss 2 | 96 °C |
| Schuss 3 | 131 °C |
| Schuss 4 | 110 °C |
| Schuss 5 | 97 °C |
| Düse | 90 °C |

Die Freisetzung nach 8 Stunden betrug 45 % [Paddlemodell nach USP (pH change)].

Beispiel 6: 520 g des Pfropfcopolymers aus Beispiel 3 wurden mit 480 g Verapamil-Hydrochlorid unter folgenden Bedingungen extrudiert und zu 1000 mg-Oblong-Tabletten kalandriert.

| | |
|---|---|
| Schuss 1 | 56 °C |
| Schuss 2 | 92 °C |
| Schuss 3 | 130 °C |
| Schuss 4 | 112 °C |
| Schuss 5 | 100 °C |
| Düse | 90 °C |

Die Freisetzung nach 8 Stunden betrug 47 % [Paddlemodell nach USP (pH change)].

## Patentansprüche

1. Verfahren zur Herstellung von festen Dosierungsformen durch Vermischen von mindestens einem polymeren Bindemittel, mindestens einem Wirkstoff und gegebenenfalls üblichen Additiven unter Bildung eines plastischen Gemisches und Formgebung, **dadurch gekennzeichnet, dass** man als polymeres Bindemittel ein wasserquellbares Pfropfcopolymerisat oder ein Gemisch derartiger Pfropfcopolymerisate einsetzt, das durch radikalisch initiierte Polymerisation von Monomeren a) ausgewählt unter
a) C₁-C₃₀-Alkylestern, Hydroxy-C₂-C₄-alkylestern, Amiden, Mono- oder Di-C₁-C₄-alkylamiden oder Nitrilen monoethylenisch ungesättigter C₃-C₃₀-Mono- oder Dicarbonsäuren oder Gemischen davon in Gegenwart von
b) sauerstoffhaltigen Polymerisaten als Pfropfgrundlage,
c) gegebenenfalls einem oder mehreren, mindestens zwei ethylenisch ungesättigte nicht-konjugierte Doppelbindungen aufweisenden Monomeren
erhältlich ist, wobei die Monomere a) bis zu 5 Gew.-%, bezogen auf die Gesamtmenge der Monomere a), einer α,β-monoethylenisch ungesättigten C₃-C₈-Carbonsäure umfassen können.

2. Verfahren nach Anspruch 1, **dadurch gekennzeichnet, dass** man als Komponente a) einem Ester der Acrylsäure oder Methacrylsäure mit einem C₁- bis C₈-Alkanol oder Mischungen davon einsetzt.

3. Verfahren nach Anspruch 1 oder 2, **dadurch gekennzeichnet, dass** man als Komponente b) Homo- oder Copolymerisate von C₂-C₄-Alkylenoxiden, insbesondere von Ethylenoxid, Propylenoxid, n-Butylenoxid oder Isobutylenoxid einsetzt.

4. Verfahren nach einem der Ansprüche bis 3, **dadurch gekennzeichnet, dass** man die Komponenten a) und b) im Gewichtsverhältnis im Bereich von 95 bis 10 : 5 bis 90, insbesondere 85 bis 55 : 15 bis 45 einsetzt.

5. Verfahren nach einem der vorhergehenden Ansprüche, **dadurch gekennzeichnet, dass** das Pfropfcopolymerisat einen K-Wert nach Fikentscher von 7 bis 100, insbesondere 10 bis 100, vorzugsweise 20 bis 100, besonders bevorzugt 20 bis 35 aufweist.

6. Verfahren nach einem der vorhergehenden Ansprüche, **dadurch gekennzeichnet, dass** die Bildung des plastischen Gemisches durch Vermischen und/oder Aufschmelzen der Komponenten in einem Extruder erfolgt.

7. Verfahren nach einem der vorhergehenden Ansprüche zur Herstellung von pharmazeutischen Dosierungsformen, Riechstoffformulierungen, Pflanzenbehandlungsmitteln, Futtermittelzusatzstoffen und -zusätzen und Nahrungsmittelzusätzen.

8. Feste Dosierungsformen, die als polymeres Bindemittel ein Pfropfcopolymerisat gemäß der Definition in einem der Ansprüche 1 bis 6 enthalten.

9. Verwendung von Pfropfcopolymerisaten gemäß der Definition in einem der Ansprüche 1 bis 5 als Bindemittel in einem Verfahren nach einem der Ansprüche 1 bis 7.

## Claims

1. A process for producing solid dosage forms by mixing at least one polymer binder, at least one active ingredient and, where appropriate, conventional additives to form a plastic mixture, and shaping, wherein a water-swellable graft copolymer or a mixture of graft copolymers is employed as polymeric binder which is obtainable by polymerization, initiated by free radicals, of monomers a) selected from
a) C₁-C₃₀-alkyl esters, hydroxy-C₂-C₄-alkyl esters, amides, mono- or di-C₁-C₄-alkylamides or nitriles of monoethylenically unsaturated C₃-C₃₀-mono- or dicarboxylic acids or mixtures thereof in the presence of
b) oxygen-containing polymers as grafting base,
c) where appropriate one or more monomers having at least two nonconjugated ethylenic double bonds,
it being possible for the monomers a) to comprise up to 5% by weight, based on the total amount of monomers a), of an α,β-monoethylenically unsaturated C₃-C₈-carboxylic acid.

2. A process as claimed in claim 1, wherein an ester of acrylic acid or methacrylic acid with a C₁-C₈-alkanol or mixtures thereof is employed as component a).

3. A process as claimed in claim 1 or 2, wherein homo- or copolymers of C₂-C₄-alkylene oxides, in particular of ethylene oxide, propylene oxide, n-butylene oxide or isobutylene oxide, are employed as component b).

4. A process as claimed in any of claims 1 to 3, wherein components a) and b) are employed in a ratio by weight in the range from 95 to 10 : 5 to 90, in particular 85 to 55 : 15 to 45.

5. A process as claimed in any of the preceding claims, wherein the graft copolymer has a Fikentscher K value of from 7 to 100, in particular 10 to 100, preferably 20 to 100, particularly preferably 20 to 35.

6. A process as claimed in any of the preceding claims, wherein the formation of the plastic mixture takes place by mixing and/or melting the components in an extruder.

7. A process as claimed in any of the preceding claims for producing pharmaceutical dosage forms, fragrance formulations, plant treatment compositions, animal feed additives and supplements and human food supplements.

8. A solid dosage form which comprises as polymeric binder a graft copolymer as defined in any of claims 1 to 6.

9. The use of graft copolymers as defined in any of claims 1 to 5 as binder in a process as claimed in any of claims 1 to 7.

## Revendications

1. Procédé pour la préparation des formes de dosage solides par mélange d'au moins un liant polymère, d'au moins une substance active et le cas échéant d'additifs usuels avec formation d'un mélange plastique qu'on soumet à façonnage, **caractérisé en ce que** l'on utilise en tant que liant polymère un copolymère greffé gonflable à l'eau ou un mélange de tels copolymères greffés, qu'on obtient par polymérisation avec induction radicalaire de monomères a) choisis parmi :
a) les esters alkyliques en C₁-C₃₀, les esters hydroxvalkyliques en C₂-C₄, les amides, les mono- ou di-(alkyle en C₁-C₄)amides ou nitriles d'acides mono- ou di-carboxyliques à insaturation monoéthylénique en C₃-C₃₀ ou leurs mélanges, en présence de
b) des polymères contenant de l'oxygène et qui constituent le support de greffage,
c) le cas échéant un ou plusieurs monomères contenant au moins deux doubles liaisons à insaturation éthylénique non conjuguées,
les monomères a) pouvant comprendre jusqu'à 5% de leur poids total d'un acide carboxylique à insaturation α,β-monoéthylénique en C₃-C₈.

2. Procédé selon la revendication 1, **caractérisé en ce que** l'on utilise en tant que composant a) un ester de l'acide acrylique ou de l'acide méthacrylique et d'un alcanol en C₁-C₈ ou un mélange de tels esters.

3. Procédé selon la revendication 1 ou 2, **caractérisé en ce que** l'on utilise pour le composant b) des homo- ou co-polymères d'oxydes d'alkylène en C₂-C₄, plus spécialement de l'oxyde d'éthylène, de l'oxyde de propylène, de l'oxyde de n-butylène ou de l'oxyde d'isobutylène.

4. Procédé selon l'une des revendications 1 à 3, **caractérisé en ce que** l'on met en oeuvre les composants a) et b) à des proportions relatives en poids dans l'intervalle de 95 à 10 : 5 à 90, plus spécialement de 85 à 55 : 15 à 45.

5. Procédé selon l'une des revendications qui précèdent, **caractérisé en ce que** le copolymère greffé a un indice K selon Fikentscher de 7 à 100, plus spécialement de 10 à 100, de préférence de 20 à 100 et dans les meilleures conditions de 20 à 35.

6. Procédé selon l'une des revendications qui précèdent, **caractérisé en ce que** le mélange plastique est formé par mélange et/ou fusion des composants dans une extrudeuse.

7. Procédé selon l'une des revendications qui précèdent, pour la préparation de formes de dosage pharmaceutiques, de compositions de parfums, de produits phytosanitaires, de compléments et additifs pour aliments du bétail et d'additifs pour produits alimentaires.

8. Formes de dosage solides contenant, en tant que liant polymère, un copolymère greffé tel que défini dans l'une des revendications 1 à 6.

9. Utilisation de copolymères greffés tels que définis dans l'une des revendications 1 à 5 en tant que liants dans un procédé selon l'une des revendications 1 à 7.
